# EUROPEAN PATENT APPLICATION

(11) **EP 4 691 392 A1**
(43) Date of publication of application: **11.02.2026**
(21) Application number: 24778155.2
(22) Date of filing: 28.03.2024
(51) Int. Cl.: A61B 17/68, A61B 17/04

(54) **ORTHOPEDIC FIXATION FASTENER**

(30) Priority: 28.03.2023 US 202363455184 P
(71) Applicant: De Novo Orthopedics Co., Ltd., Taichung (TW)
(72) Inventor: LIN, Chiawei, Taichung City Taiwan (CN); TAI, Tawei, Tainan City Taiwan (CN)
(74) Representative: Müller Hoffmann & Partner
(86) International application number: PCT/CN2024/084469
(87) International publication number: WO 2024/199369

(57) **Abstract**

The present disclosure provides an orthopedic fixation fastener for securing a suture, including a fixation fastener body (1) and a fixator (2). The fixation fastener body (1) includes a central portion (11), a first fixing portion (12), and a second fixing portion (13), which are correspondingly connected to opposite sides of the central portion (11). Each of the first fixing portion (12) and second fixing portion (13) includes a suture hole (121, 131) extending through a top surface (14) and a bottom surface (15), through which the suture passes. The fixator (2) includes a fixing structure (21) detachably connectable to the fixation fastener body (1), such that when the fixator (2) is engaged, the suture is accommodated and fixed between the fixator (2) and the fixation fastener body (1) without requiring additional knotting for fixation.

## Description

### FIELD

The present disclosure generally relates to an orthopedic fixation fastener for securing a suture, and, more particularly, to an orthopedic fixation fastener for tightly securing a suture to affected tissues without the need for knotting.

### BACKGROUND

Orthopedic fixation fasteners are commonly used to support or fix bone fragments, tendon rupture sites, or both, to promote stable healing between bone and surrounding soft tissues. When using an orthopedic fixation fastener, it is necessary to tighten the suture and tie a knot to apply compressive force on the tissue and prevent suture slippage. However, the effectiveness of the orthopedic fixation fastener is dependent on knot quality, which in turn would affect the surgical outcome.

### SUMMARY

In view of the above, to address the limitations of prior art orthopedic fixation fasteners, the present disclosure provides an orthopedic fixation fastener that securely fixes a suture to affected tissues without requiring knotting.

The present disclosure provides an orthopedic fixation fastener for securing a suture, including: a fixation fastener body including a central portion, a first fixing portion, and a second fixing portion, the first and second fixing portions being connected to opposite sides of the central portion. The central portion, first fixing portion, and second fixing portion as a whole define a top surface and a bottom surface, and each of the first and second fixing portions includes a first suture hole and a second suture hole extending through the top and bottom surfaces for the suture to pass through. The orthopedic fixation fastener further includes a fixator, including a fixing structure detachably connected to the fixation fastener body, such that when the fixator is engaged with the fixation fastener body, at least one suture is compressed and fixed between them.

In some implementations, the fixing structure may include a first hook structure bent at a first angle toward the fixation fastener body, to be detachably hooked and fixed to an engaging structure in the central portion.

In some implementations, the engaging structure may be one of the sides of the central portion that is not connected to the first fixing portion and the second fixing portion.

In some implementations, the engaging structure may be a groove structure in the fixation fastener body.

In some implementations, the fixing structure may include a first hook structure bent at a first angle toward the fixation fastener body and a second hook structure bent at a second angle, to be correspondingly and detachably hooked and fixed to a set of engaging structures in the central portion. The first angle may be greater than the second angle.

In some implementations, the set of engaging structures may be the opposite sides of the central portion that are not connected to the first fixing portion and the second fixing portion.

In some implementations, the set of engaging structures may include one sides of the central portion that is not connected to the first fixing portion and the second fixing portion, together with a groove structure in the fixation fastener body.

In some implementations, when the fixator is connected to the fixation fastener body, an accommodating space having a width less than or equal to the diameter of the at least one suture may be formed between the fixator and the fixation fastener body.

In some implementations, the fixing structure may include a plurality of post structures, and the fixation fastener body may further include a plurality of corresponding hole structures, wherein the plurality of post structures may be detachably engaged with the plurality of corresponding hole structures.

In some implementations, the number of hole structures may be equal to or greater than the number of post structures, and the position of the fixator on the fixation fastener body may be determined by which of the hole structures are engaged with the post structures.

In some implementations, the fixator may further include a third suture hole for the corresponding suture to pass through.

In some implementations, a part or an entirety of the central portion of the fixation fastener body may be recessed from the top surface toward the bottom surface by a depth equal to or less than the diameter of the at least one suture, forming a recessed structure.

In some implementations, the fixing structure may include a first hook structure bent at a first angle toward the fixation fastener body, to be detachably hooked and fixed to an engaging structure in the central portion. The fixator may further include a baffle structure, the baffle structure being a protrusion extending toward the fixation fastener body by a length that is less than the diameter of the at least one suture, and disposed on a side of the central portion that is not connected to the first fixing portion and the second fixing portion when the fixator is engaged.

In some implementations, the fixing structure may include a plurality of post structures, and the fixation fastener body may further include a plurality of corresponding and detachably engageable hole structures. The fixator may further include a baffle structure, the baffle structure being a protrusion extending from the fixator toward the fixation fastener body by a length that is less than the diameter of the at least one suture, and disposed on a side of the central portion that is not connected to the first fixing portion and the second fixing portion when the fixator is engaged.

In some implementations, a part of the recessed structure may form a directional groove for accommodating the at least one suture.

In some implementations, the orthopedic fixation fastener may further include a removal structure, which may be disposed on a side edge of the fixation fastener body where the fixation fastener body connects to the fixator, or on a side edge of the fixator where the fixator connects to the fixation fastener body.

The orthopedic fixation fastener of the present disclosure includes a fixation fastener body for the suture to pass through and apply uniform pressure on tissue, and a fixator detachably connected to the fixation fastener body, such that the suture is compressed and secured between them without additional knotting to prevent slippage. The fixation fastener of the present disclosure may be substantially plate-shaped to distribute pressure evenly over a large surface area, reducing uneven tissue growth or remodeling caused by stress concentrations. Furthermore, since the fixator is detachably connected to the fixation fastener body, when using the orthopedic fixation fastener of the present disclosure to fix a suture, the orthopedic fixation fastener may still be detached for adjustment after the fixation fastener body and the fixator are assembled together.

### BRIEF DESCRIPTION OF THE DRAWINGS

The present disclosure will be better understood from the following detailed description when read in conjunction with the accompanying drawings, in which:
FIG. 1 is a top view of an orthopedic fixation fastener according to one implementation of the present disclosure;
FIG. 2 is an exploded view of the orthopedic fixation fastener according to one implementation of the present disclosure;
FIG. 3 is a top view of a fixation fastener body according to one implementation of the present disclosure;
FIG. 4 is a side view of the fixation fastener body taken along a line A-A of FIG. 3;
FIG. 5 is a top view of a fixator according to one implementation of the present disclosure;
FIG. 6 is a side view of the fixator taken along a line B-B of FIG. 5;
FIG. 7 is a top view of an orthopedic fixation fastener according to another implementation of the present disclosure;
FIG. 8A is an exploded view of an orthopedic fixation fastener according to another implementation of the present disclosure;
FIG. 8B is a partial side view of the orthopedic fixation fastener according to another implementation of the present disclosure;
FIG. 9 is a perspective view of a fixator according to another implementation of the present disclosure;
FIG. 10 is an exploded view of an orthopedic fixation fastener according to another implementation of the present disclosure;
FIG. 11 is a perspective view of a fixator according to another implementation of the present disclosure; and
FIG. 12 is a perspective view of a fixator according to another implementation of the present disclosure.

### DETAILED DESCRIPTION

The following description provides specific information regarding exemplary implementations of the present disclosure. The drawings and the accompanying detailed description are directed to such exemplary implementations. However, the present disclosure is not limited thereto. Those of ordinary skill in the art will readily appreciate other variations and modifications within the scope of the present disclosure. Unless otherwise specified, identical or corresponding elements in the drawings may be designated by the same reference numerals. Furthermore, the drawings and illustrations of the present disclosure are generally not to scale and are not intended to represent actual relative dimensions.

For clarity and ease of understanding, reference numerals are used in the exemplary drawings to identify corresponding features (although not indicated in some examples). However, features in different implementations may vary in other respects and should not be narrowly construed as being limited to those shown in the drawings.

The expressions "at least one implementation," "an implementation," "multiple implementations," "different implementations," "some implementations," "the present implementation," and similar terms are used to indicate that an implementation of the present disclosure may include a particular feature, structure, or characteristic, but not every implementation must necessarily include such feature, structure, or characteristic. Furthermore, repeated use of the phrases "in one implementation" or "in the present implementation" does not necessarily refer to the same implementation, although they may. In addition, references to "implementation" in association with "the present disclosure" should be understood as meaning "at least some implementations of the present disclosure" include the described feature, structure, or characteristic, rather than implying that all implementations must include it.

The terms "first," "second," "third," and the like in the description and drawings of the present disclosure are merely used to distinguish between different elements and are not intended to denote a specific order. In addition, the term "connected," as used in the description and drawings of the present disclosure, refers to either a direct or indirect connection through intermediate elements, and is not necessarily limited to a physical connection.

The term "including" and variations thereof, as used in the description and drawings of the present disclosure, are intended to cover a non-exclusive inclusion, meaning "including but not limited to." This indicates an open-ended relationship for the recited combinations, groups, series, and equivalents. For example, a process, method, system, product, or device including a series of steps or modules is not limited to those expressly listed, but may additionally include steps or modules not listed, or other steps or modules inherent to such processes, methods, products, or devices.

For the purpose of explanation, and without limitation, specific details such as functional entities, technologies, protocols, and standards are set forth to facilitate understanding of the described technology. However, detailed descriptions of well-known methods, techniques, systems, or architectures are omitted to avoid obscuring the disclosure with unnecessary detail.

The present disclosure generally relates to an orthopedic fixation fastener for fixing a suture, and more particularly to an orthopedic fixation fastener for securing a suture to affected tissues without the need for knotting. The orthopedic fixation fastener of the present disclosure includes a fixation fastener body through which the suture passes to apply uniform pressure on tissues, and a fixator detachably connected to the fixation fastener body, such that the suture is compressed and secured between them without knotting, so as to preventing slippage. The fixation fastener of the present disclosure may be substantially plate-shaped to distribute pressure evenly over a larger surface area, so as to reduce uneven tissue growth or remodeling caused by localized stress concentrations. Furthermore, since the fixator is detachably connected to the fixation fastener body, the orthopedic fixation fastener may be disassembled and adjusted even after the fixation fastener body and fixator have been assembled together.

For example, the orthopedic fixation fastener of the present disclosure may be applied in a tendon suture surgery, although it is not limited to such use. More specifically, in a tendon suture surgery, when suturing a ruptured tendon to bone, a suture anchor is typically inserted into the bone to fix the suture and pull the tendon and bone tightly together. To prevent tendon slippage along the suture, knots are conventionally tied after the suture passes through the tendon. The orthopedic fixation fastener of the present disclosure may replace this conventional knotting method by pressing and fixing the suture of the suture anchor onto the tendon surface, so as to provide stable pressure to press the tendon against the bone and prevent suture slippage that could compromise the surgical outcome. Accordingly, the orthopedic fixation fastener of the present disclosure not only eliminates the step of knotting during surgery but also reduces the risk of poor prognosis caused by uneven surface pressure or knotting quality issues.

The following provides a more detailed description of the present disclosure with reference to the accompanying drawings and implementations.

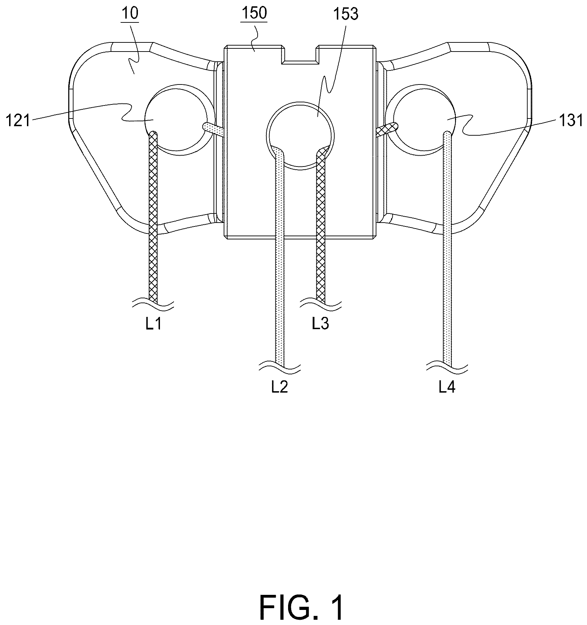

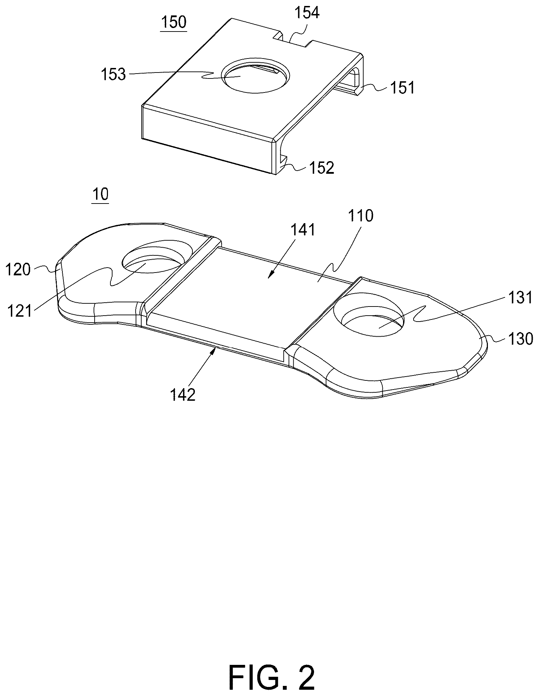

Referring to FIG. 1, a top view of an orthopedic fixation fastener according to one implementation of the present disclosure is illustrated. Referring also to FIG. 2, an exploded view of the orthopedic fixation fastener according to one implementation of the present disclosure is illustrated.

In some implementations, the orthopedic fixation fastener of the present disclosure includes a fixation fastener body 10 through which a plurality of sutures (L1-L4) passes to apply uniform pressure on tissues, and a fixator 150 detachably connected to the fixation fastener body 10. When the fixator 150 is connected to the fixation fastener body 10, one or more of the plurality of sutures (L1-L4) are compressed and fixed between the fixator 150 and the fixation fastener body 10. By way of example, in one type of tendon suture surgery, as shown in FIG. 1, at least sutures L2 and L3 may be compressed and fixed between the fixator 150 and the fixation fastener body 10.

In some implementations, the fixation fastener body 10 may be substantially plate-shaped and include a central portion 110, and a first fixing portion 120 and a second fixing portion 130 connected respectively to opposite sides of the central portion 110. The central portion 110, the first fixing portion 120, and the second fixing portion 130 together define a top surface 141 and a bottom surface 142. Each of the first fixing portion 120 and the second fixing portion 130 may include a first suture hole 121 and a second suture hole 131 extending through the top surface 141 and the bottom surface 142, for the plurality of sutures (L1-L4) to pass through.

In some implementations, the fixator 150 may also be substantially plate-shaped and include a fixing structure detachably connected to the central portion 110 of the fixation fastener body 10. In some implementations, the surface formed by the substantially plate-shaped fixator 150 may be regarded as a horizontal plane. The fixing structure may include a first hook structure 151 bent toward the fixation fastener body 10 along one edge of the horizontal plane to be detachably hooked and fixed to an engaging structure in the central portion 110. For example, the engaging structure may be one of the sides of the central portion 110 that is not connected to the first fixing portion 120 and the second fixing portion 130, or a groove structure in the fixation fastener body 10. In some implementations, the surface formed by the substantially plate-shaped fixator 150 may be defined as a horizontal plane, and the fixing structure may be a first hook structure 151 bent toward the fixation fastener body 10 along one edge of the horizontal plane, and a second hook structure 152 bent toward the fixation fastener body 10 along another edge of the horizontal plane, to be detachably hooked and fixed to a set of engaging structures in the central portion 110. For example, the set of engaging structures may be the opposite sides of the central portion 110 that are not connected to the first fixing portion 120 and the second fixing portion 130, one of the sides of the central portion 110 not connected to the first and second fixing portions in combination with a groove structure in the fixation fastener body 10, or two or more groove structures in the fixation fastener body 10.

In some implementations, the substantially plate-shaped fixator 150 may define a third suture hole 153 extending through the top and bottom surfaces for the plurality of sutures (L1-L4) to pass through. In some implementations, the third suture hole 153 may be positioned approximately at the center of the plate-shaped surface, which enables a user to more conveniently pass the sutures through during the use of the orthopedic fixation fastener of the present disclosure. This arrangement may also help mitigate the problem of prolonged assembly time that could otherwise arise due to the compact overall structure.

In some implementations, the surface formed by the substantially plate-shaped fixator 150 may be defined as a horizontal plane, and a removal groove 154 may be formed along one side edge of the horizontal plane. The removal groove 154 provides a space for a tool to apply force when detaching the fixator 150 from the fixation fastener body 10, and the removal groove 154 may also serve as a positioning feature during assembly. More specifically, the removal groove 154 may be disposed on the edge of the horizontal plane adjacent to the first fixing portion 120 or the second fixing portion 130.

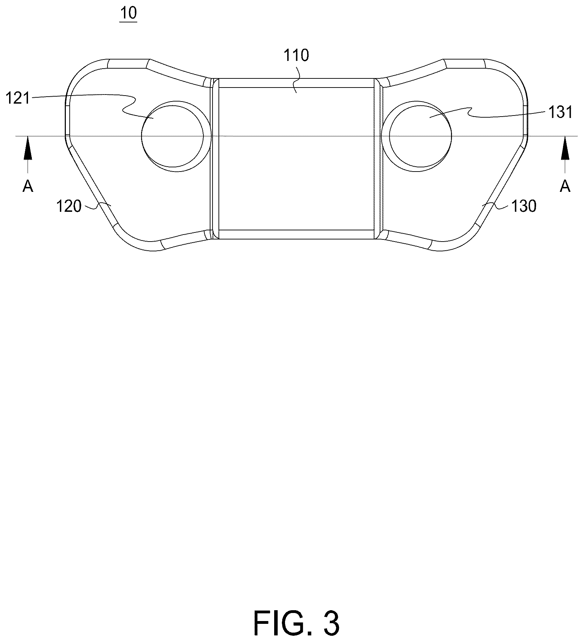

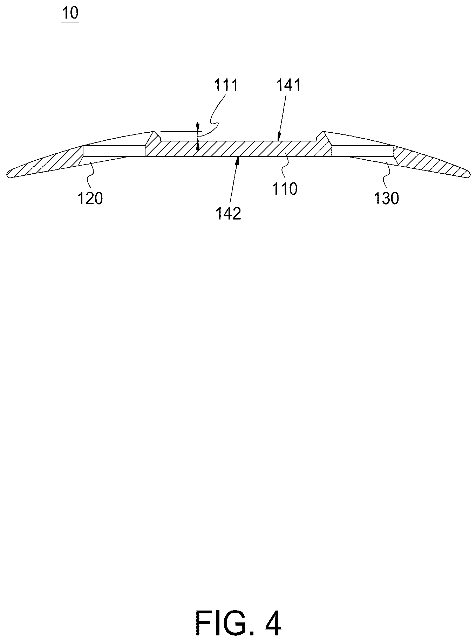

Referring to FIG. 3 and FIG. 4, which respectively illustrate a top view of the fixation fastener body and a side view taken along a line A-A, according to one implementation of the present disclosure. In some implementations, the fixation fastener body 10 may be substantially plate-shaped, with the plane formed by the central portion 110 defined as a horizontal plane. From a side view, the first fixing portion 120 may be slightly inclined counterclockwise relative to the horizontal plane to form a first plane, and the second fixing portion 130 may be slightly inclined clockwise relative to the horizontal plane to form a second plane. In this configuration, the top surface 141 may form a curved surface to conform to the geometry of hard tissues, while the bottom surface 142 may include a central horizontal plane flanked by inclined planes on opposite sides to conform to the geometry of soft tissues. The inclination angles of the planes formed by the first fixing portion 120 and the second fixing portion 130 may be adjusted depending on specific application scenarios.

In some implementations, the central portion 110 of the fixation fastener body 10 may have the greatest thickness so as to enhance overall rigidity and prevent fracture. In some implementations, the central portion 110 may form a recessed structure 111 that is slightly recessed from the top surface 141 toward the bottom surface 142 to accommodate one or more of the plurality of sutures (L1-L4). The recessed structure 111 may be at least partially covered by the fixator 150 so that the sutures accommodated therein may be secured between the fixation fastener body 10 and the fixator 150. In some implementations, the recessed depth of the recessed structure 111 may be equal to or slightly less than the diameter of the plurality of sutures (L1-L4), for example about 0.24-3.6 mm (preferably 0.3 mm), so as to provide a receiving space for one or more of the plurality of sutures (L1-L4) while increase the compressive force exerted by the fixator 150 on the sutures within the recessed structure 111. In some implementations, the central portion 110 of the fixation fastener body 10 may not form the recessed structure 111 that is slightly recessed from the top surface 142 toward the bottom surface 143.

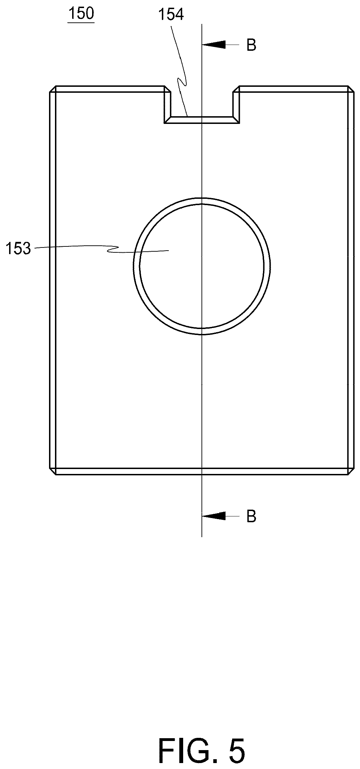

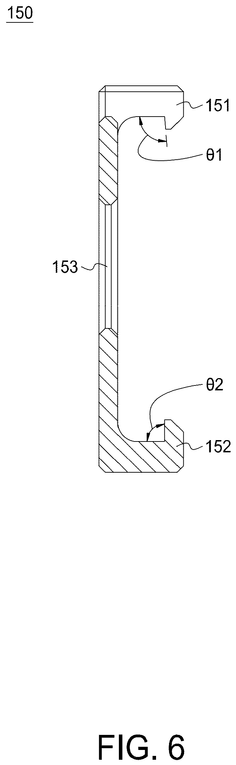

Referring to FIG. 5 and FIG. 6, which respectively illustrate a top view of the fixator and a side view taken along a line B-B, according to one implementation of the present disclosure. In some implementations, the fixation fastener body 10 may be substantially plate-shaped, and the fixator 150 may likewise be substantially plate-shaped. A first hook structure 151 and a second hook structure 152 may be formed corresponding to the sides of the fixation fastener body 10, so as to be hooked and secured onto opposite sides of the fixation fastener body 10. For example, when the side of the fixation fastener body 10 is a rectangular structure, the first hook structure 151 and the second hook structure 152 may be formed by extending a predetermined length from two opposite edges of the horizontal plane of the fixator 150 toward the fixation fastener body 10 and then bending at a first angle θ1 and a second angle θ2, respectively, so as to form a frame-like structure capable of hooking onto the rectangular side structure.

In some preferred implementations, the first angle θ1 may be greater than the second angle θ2. Thus, when adjustment of the suture is required during use of the orthopedic fixation fastener of the present disclosure, the first hook structure 151 may be pried open with relatively less force, so as to allow the fixator 150 to be easily detached. In some preferred implementations, the second angle θ2 may be approximately 90°, such that the second hook structure 152 may be securely hooked to the side of the fixation fastener body 10, while the first angle θ1 may be slightly greater than the second angle θ2, for example about 95° or 100°. In this manner, the fixator 150 may be conveniently detached for suture adjustment, while still maintaining sufficient hooking and fixing capability.

In some implementations, as shown in FIG. 2 or FIG. 4, when the recessed structure 111 is formed in the central portion 110 of the fixation fastener body 10, the extension length of the two opposite edges of the horizontal plane of the fixator 150 toward the fixation fastener body 10 may be substantially equal to the side length of the fixation fastener body 10. This ensures that the fixator 150 applies sufficient compressive force to the sutures contained therein. Alternatively, the recessed depth of the recessed structure 111 may be adjusted so that the difference between the extension length and the side length of the fixation fastener body 10, combined with the recessed depth, is equal to or slightly less than the diameter of the plurality of sutures (L1-L4).

Alternatively, in some implementations, the central portion 110 of the fixation fastener body 10 may not include the recessed structure 111, and an accommodating space for one or more of the plurality of sutures (L1-L4) may be formed in other ways. For example, when the central portion 110 does not include the recessed structure 111, the extension length of the two opposite edges of the horizontal plane of the fixator 150 toward the fixation fastener body 10 may be slightly greater than the side length of the fixation fastener body 10. That is, when both the first hook structure 151 and the second hook structure 152 are hooked onto the sides of the fixation fastener body 10, an accommodating space for one or more of the plurality of sutures (L1-L4) may be formed between the fixator 150 and the fixation fastener body 10. Preferably, the difference between the extension length and the side length of the fixation fastener body 10 may be equal to or slightly less than the diameter of the plurality of sutures (L1-L4), so as to increase the compressive force exerted by the fixator 150 on the sutures contained therein.

Referring to FIGS. 1 to 6, a tendon suturing procedure requiring two suture anchors is taken as an example to illustrate how the orthopedic fixation fastener of the present disclosure secures sutures. In this example, a total of four sutures (L1-L4) pass through the tendon to be sutured, wherein sutures L1 and L2 are located on a first side near the first suture hole 121, and sutures L3 and L4 are located on a second side near the second suture hole 131. The user first provides the fixation fastener body 10 and passes the sutures L1 and L2 through the first suture hole 121, and the sutures L3 and L4 through the second suture hole 131. The fixation fastener body 10 is then pressed firmly against the tendon tissue, such that the pressure is evenly distributed over a larger surface area, so as to reduce the likelihood of tissue proliferation or irregular remodeling caused by uneven loading. Next, the second hook structure 152 of the fixator 150 is hooked onto one side edge of the fixation fastener body 10, after which the sutures L2 and L3 are drawn toward the central portion 110 of the fixation fastener body 10 and passed through the third suture hole 153. The four sutures (L1-L4) are then tightened and properly positioned, and the first hook structure 151 of the fixator 150 is latched to hook onto the opposite side edge of the fixation fastener body 10. In this way, the sutures are compressed and secured without the need for knotting. Furthermore, due to the structural design of the first hook structure 151 and the second hook structure 152, if further adjustment is required after assembly of the orthopedic fixation fastener, the user may simply pry the removal groove 154 with a tool to easily separate the fixation fastener body 10 from the fixator 150, so as to greatly enhance usability.

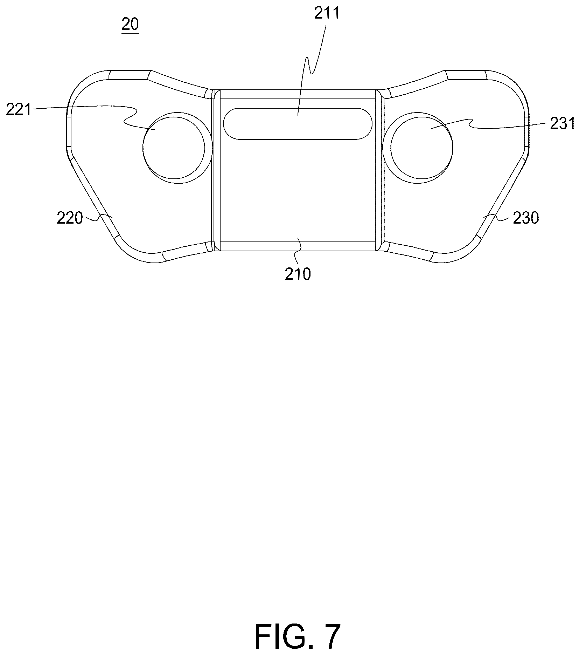

Referring to FIG. 7, which is a top view of an orthopedic fixation fastener according to another implementation of the present disclosure. In some implementations, the fixation fastener body 20 may be substantially plate-shaped and include a central portion 210, with a first fixing portion 220 and a second fixing portion 230 respectively connected to opposite sides of the central portion 210. Each of the first fixing portion 220 and the second fixing portion 230 may include a first suture hole 221 and a second suture hole 231 extending through the top and bottom surfaces for sutures to pass through.

In this implementation, the fixation fastener body 20 has substantially the same structure as the aforementioned fixation fastener body 10 and may also be used with the fixator 150. The only difference is that the central portion 210 additionally includes an elongated groove 211, for example, an oval groove, into which the first hook structure 151 of the fixator 150 may be tightly hooked. The long side of the elongated groove 211 may be less than or approximately equal to the width of the central portion 210 to provide an alignment function for positioning engagement. The elongated groove 211 may or may not extend through the top and bottom surfaces. In some preferred implementations, the elongated groove 211 may be disposed near the edge of the central portion 210 to increase the space for accommodating sutures between the fixation fastener body 20 and the fixator 150. In some implementations, the elongated groove 211 may be disposed on one or both sides of the central portion 210, regardless of whether the sides are connected to the first and second fixing portions.

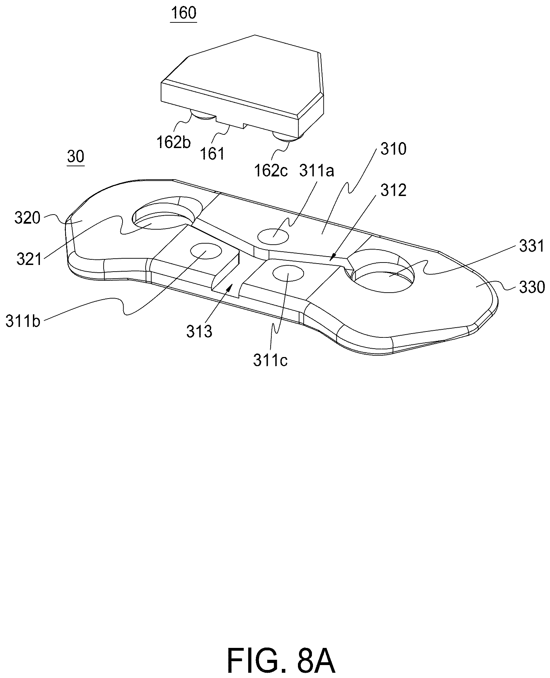

Referring to FIG. 8A, which is an exploded view of an orthopedic fixation fastener according to another implementation of the present disclosure. In some implementations, the fixation fastener body 30 may be substantially plate-shaped and include a central portion 310, with a first fixing portion 320 and a second fixing portion 330 respectively connected to opposite sides of the central portion 310. Each of the first fixing portion 320 and the second fixing portion 330 may include a first suture hole 321 and a second suture hole 331 extending through the top and bottom surfaces for sutures to pass through.

In some implementations, the fixator 160 may also be substantially plate-shaped and include a fixing structure configured to be detachably connected to the central portion 310 of the fixation fastener body 30. In some implementations, the fixing structure may include a plurality of post structures (162a-162c), and the fixation fastener body 30 may further include a plurality of detachably engageable hole structures (311a-311c) corresponding to the plurality of post structures (162a-162c). The number and arrangement of the post structures (162a-162c) are not limited and may be adjusted according to different application scenarios. In some implementations, the number of hole structures may be equal to or greater than the number of post structures. In some preferred implementations, the number of hole structures may be greater than the number of post structures, such that the post structures may be selectively engaged with different hole structures, so as to determine the position of the fixator relative to the fixation fastener body.

In this implementation, the fixation fastener body 30 has substantially the same structure as the aforementioned fixation fastener body 10, with the only difference being that the central portion 310 includes a plurality of hole structures (311a-311c). The fixator 160 may be connected to the fixation fastener body 30 by engaging the plurality of post structures (162a-162c) with the plurality of hole structures (311a-311c), such that one or more of the plurality of sutures are compressed and fixed between the fixator 160 and the fixation fastener body 30.

In some implementations, the surfaces of the plurality of post structures (162a-162c) may include a reinforcing structure to stabilize the connection strength between the fixator 160 and the fixation fastener body 30 when loosely engaged with the plurality of hole structures (311a-311c). For example, an annular protrusion may be provided at the end of each post structure. In some implementations, the reinforcing structure or another strengthening structure on the surfaces of the plurality of post structures (162a-162c) may stabilize the connection strength between the fixator 160 and the fixation fastener body 30 when fully engaged with the plurality of hole structures (311a-311c). For example, an annular protrusion may be provided at the end of each post structure.

In some implementations, the central portion 310 of the fixation fastener body 30 may also form a recessed structure as described previously to accommodate one or more of the plurality of sutures. The recessed structure may at least be partially covered by the fixator 160 to secure the sutures contained between the fixation fastener body 30 and the fixator 160. In some implementations, the recessed structure may be a directional groove 312, with its two ends respectively connected to the first suture hole 321 and the second suture hole 331. Another elongated directional groove 313 may extend from approximately the center of the directional groove 312 to a side edge of the central portion 310, so as to guide the path of the sutures in the central portion 310 to provide a positioning function.

In some implementations, the surface defined by the substantially plate-shaped fixator 160 may be regarded as a horizontal plane, and a baffle structure 161 may extend from one edge of this horizontal plane toward the fixation fastener body 30, corresponding to the elongated directional groove 313, to compress the sutures contained within the elongated directional groove 313. This arrangement increases the compressive force of the fixator 160 on the sutures contained in the recessed structure. In some implementations, the side of the baffle structure 161 that contacts the sutures may be roughened or serrated to increase friction.

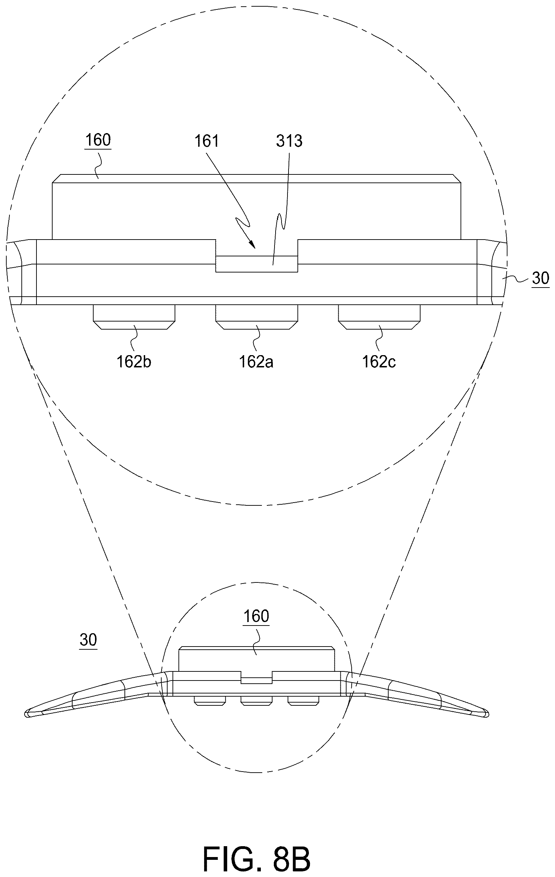

To further clarify this implementation, a tendon suture surgery requiring two suture anchors is used as an example to illustrate the method of fixing sutures using the orthopedic fixation fastener of the present disclosure. Referring also to FIG. 8B, which is a partial side view of an orthopedic fixation fastener according to another implementation of the present disclosure. A total of four sutures (L1-L4) passes through the tendon to be sutured, with sutures L1 and L2 on a first side and sutures L3 and L4 on a second side. The user first provides the fixation fastener body 30 and passes the sutures L1 and L2 through the first suture hole 321 and the sutures L3 and L4 through the second suture hole 331. Next, the fixation fastener body 30 is pressed tightly against the tendon tissue to distribute pressure evenly over a larger surface area, thereby reducing uneven tissue hyperplasia or remodeling caused by uneven stress. Then, the plurality of post structures (162a-162c) of the fixator 160 is loosely engaged with the plurality of hole structures (311a-311c) in the central portion 310 of the fixation fastener body 30. The sutures L2 and L3 are pulled toward the central portion 310, with the suture L2 guided along the directional groove 312 and then along the elongated directional groove 313 to the edge of the central portion 310, and the suture L3 is similarly guided along the directional groove 312 and the elongated directional groove 313 to the edge of the central portion 310. The four sutures (L1-L4) are then tightened and adjusted to the proper position, and the plurality of post structures (162a-162c) is fully engaged in the plurality of hole structures (311a-311c). This compresses and secures the sutures to prevent slippage without the step of tying a knot, thereby greatly improving ease of use.

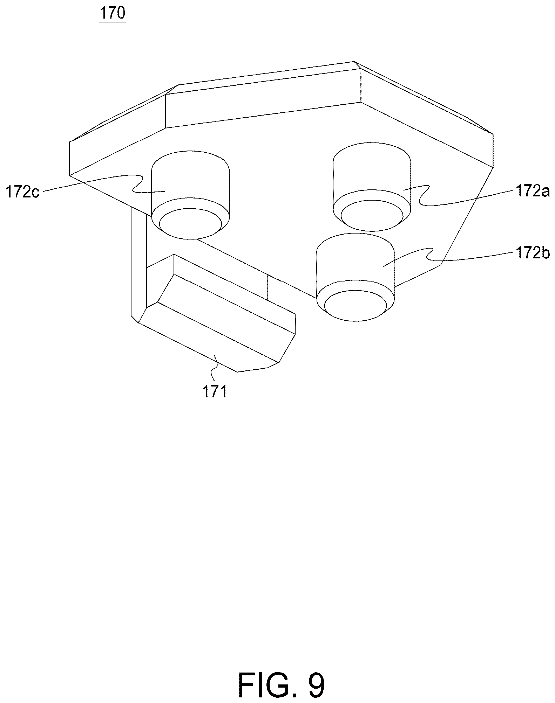

Referring to FIG. 9, which is a perspective view of a fixator according to another implementation of the present disclosure. In some implementations, the fixator 170 has substantially the same structure as the aforementioned fixator 160, particularly in that the fixing structure may be a plurality of post structures (172a-172c) configured to be used with the fixation fastener body 30. The difference is that the fixing structure of the fixator 170 may further include a first hook structure 171, which is bent from one edge of the plate-shaped surface toward the fixation fastener body 30 and may be detachably hooked and fixed to one of the side surfaces of the central portion 310 that is not connected to the first fixing portion 320 and the second fixing portion 330, so as to strengthen the connection of the fixator 170 to the fixation fastener body 30. In some implementations, the fixator 170 may also be used with a fixation fastener body, whose central portion does not form a recessed structure.

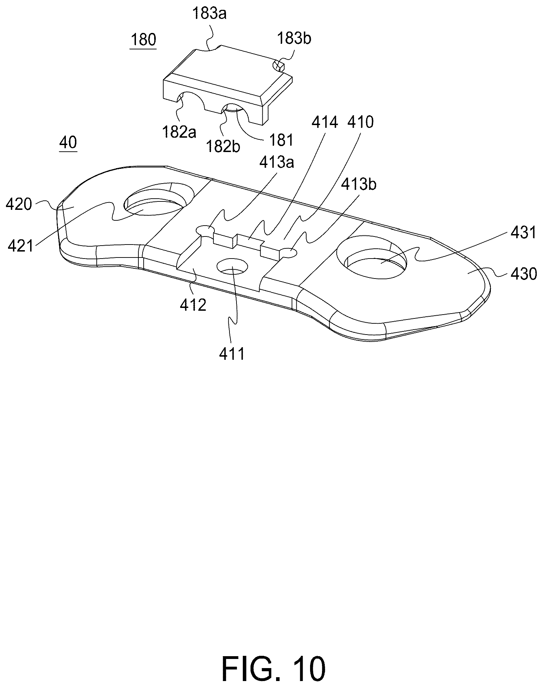

Referring to FIG. 10, which is an exploded view of an orthopedic fixation fastener according to another implementation of the present disclosure. In some implementations, the fixation fastener body 40 of the orthopedic fixation fastener may be substantially plate-shaped and include a central portion 410, with a first fixing portion 420 and a second fixing portion 430 respectively connected to opposite sides of the central portion 410. Each of the first fixing portion 420 and the second fixing portion 430 may include a first suture hole 421 and a second suture hole 431 extending through the top and bottom surfaces, through which a plurality of sutures may pass.

In some implementations, the fixator 180 may also be substantially plate-shaped and include a fixing structure configured to be detachably connected to the central portion 410 of the fixation fastener body 40. The fixing structure may be a plurality of post structures 181, and the fixation fastener body 40 may further include a plurality of detachably engageable hole structures 411 corresponding the plurality of post structures 181.

In this implementation, the fixation fastener body 40 has substantially the same structure as the aforementioned fixation fastener body 30. The only difference is that the recessed structure formed in the central portion 410 of the fixation fastener body 40 may be a first directional groove 413a and a second directional groove 413b that are not connected to the first suture hole 321 and the second suture hole 331. The first directional groove 413a and the second directional groove 413b may be connected to another elongated directional groove 412, and the other end of this elongated directional groove 412 may connect to a side edge of the central portion 410, so as to guide the path of the plurality of sutures in the central portion 410 to provide a positioning function.

In some implementations, the elongated directional groove 412 of the fixation fastener body 40 may further form a removal groove 414 on the side opposite to the edge of the central portion 410 to provide a space, in which a force may be applied by a tool, when detaching the fixator 180 from the fixation fastener body 40. The removal groove 414 may also provide a positioning function during assembly. Alternatively, in some implementations, the removal groove 414 may be formed on one side edge of the horizontal plane of the fixator 180, similar to the aforementioned removal groove 154.

In some implementations, the surface of the substantially plate-shaped fixator 180 may be defined as a horizontal plane. On this horizontal plane, a first groove 183a and a second groove 183b may be formed corresponding to the first directional groove 413a and the second directional groove 413b, respectively. Along one edge of the horizontal plane, a baffle structure having a first directional hole 182a and a second directional hole 182b may extend toward the fixation fastener body 40, corresponding to the elongated directional groove 412, so as to guide the path of the plurality of sutures in the central portion 410 and provide a positioning function. The first directional hole 182a and the second directional hole 182b may collectively function as a third suture hole.

To further clarify this implementation, a tendon suture surgery requiring two suture anchors is used as an example to illustrate the method of fixing sutures using the orthopedic fixation fastener of the present disclosure. A total of four sutures (L1-L4) passes through the tendon to be sutured, with sutures L1 and L2 on a first side and sutures L3 and L4 on a second side. The user first provides the fixation fastener body 40 and passes the sutures L1 and L2 through the first suture hole 421 and the sutures L3 and L4 through the second suture hole 431. Next, the fixation fastener body 40 is pressed tightly against the tendon tissue to distribute pressure evenly over a larger surface area, thereby reducing uneven tissue hyperplasia or remodeling caused by uneven stress. The plurality of post structures 181 of the fixator 180 is then loosely engaged with the plurality of hole structures 411 in the central portion 410 of the fixation fastener body 40. The sutures L2 and L3 are pulled toward the central portion 410, with the suture L2 first passed through the through-hole that is formed by the first directional groove 413a and the first groove 183a, and then through the second directional hole 182b. Simultaneously, the suture L3 is first passed through the through-hole that is formed by the second directional groove 413b and the second groove 183b, and then through the first directional hole 182a, such that the sutures L2 and L3 are guided along the elongated directional groove 412 to the edge of the central portion 410. The four sutures (L1-L4) are then tightened and adjusted to the proper position, and the plurality of post structures 181 is fully engaged in the plurality of hole structures 411. This arrangement compresses and secures the sutures to prevent slippage without the step of tying a knot, and the fixation fastener body 40 and the fixator 180 may be easily separated by prying the removal groove 414 with a tool, so as to greatly improve ease of use.

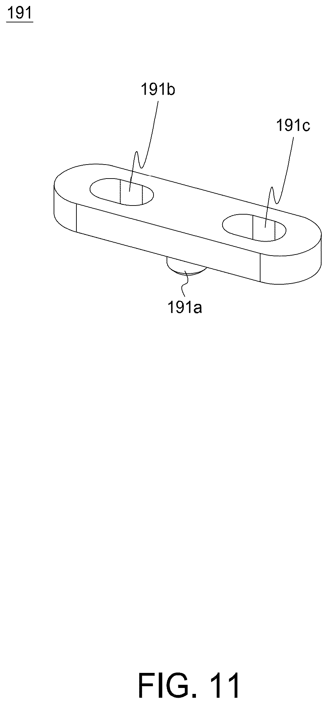

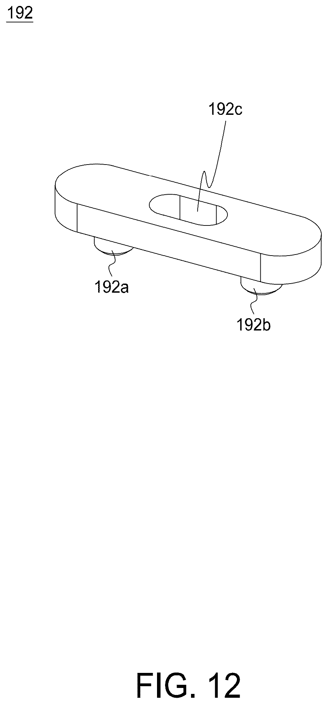

Referring to FIG. 11 and FIG. 12, which are perspective views of fixators according to another implementation of the present disclosure. In some implementations, the fixator may be substantially in the shape of an elongated plate. As shown in FIG. 11, the fixator 191 may include a post structure 191a for engaging with the fixation fastener body, and third suture holes (191b, 191c) extending through the top and bottom surfaces for one or more sutures to pass through. As shown in FIG. 12, the fixator 192 may include post structures (192a, 192b) for engaging with the fixation fastener body, and a third suture hole 192c extending through the top and bottom surfaces for a plurality of sutures to pass through.

In the orthopedic fixation fastener of the present disclosure, the fixation fastener body and the fixator are preferably made of a biocompatible material, such as polyetheretherketone (PEEK), and the fixation fastener body preferably has a smooth surface. In some implementations, the length of the fixation fastener body ranges from 5-50 mm, and may be specifically set to, for example, 10, 11, 12, 13, 14, 15, 16, 17, 18, 19, 20, 25, 30, 35, or 40 mm. In some implementations, the width of the fixation fastener body ranges from 3-35 mm, and may be specifically set to, for example, 4, 5, 6, 7, 8, 9, 10, 11, 12, 15, 20, 25, or 30 mm. In some implementations, the thickness of the central portion of the fixation fastener body ranges from 0.3-1.5 mm, and may be specifically set to, for example, 0.5, 0.6, 0.7, 0.8, 0.9, 1.0, 1.1, 1.2, or 1.3 mm, while the thickness of the first fixing portion and the second fixing portion of the fixation fastener body ranges from 0.3-1.5 mm, and may be specifically set to, for example, about 0.3, 0.4, 0.5, 0.6, 0.7, 0.8, or 0.9 mm. The dimensions of the fixator may be adjusted according to the shape design of its implementation and the dimensions of the fixation fastener body with which it is used.

The orthopedic fixation fastener of the present disclosure includes a fixation fastener body through which one or more sutures pass to apply uniform pressure on tissues, and a fixator detachably connected to the fixation fastener body, such that the suture is compressed and secured between them without additional knotting to prevent slippage. The orthopedic fixation fastener of the present disclosure may be substantially plate-shaped to distribute pressure evenly over a large surface area, so as to reduce uneven tissue hyperplasia or remodeling caused by uneven stress. Furthermore, since the fixator is detachably connected to the fixation fastener body, when using the orthopedic fixation fastener of the present disclosure to fix a suture, the orthopedic fixation fastener may still be detached for adjustment after the fixation fastener body and the fixator are assembled together.

Based on the foregoing description, various techniques may be employed to implement the concepts disclosed herein without departing from the scope of the concepts. Although the concepts have been described with reference to specific implementations, modifications in form, detail, and arrangement may be made without departing from the scope of the disclosure. The described implementations are to be regarded in all respects as illustrative rather than restrictive. The present disclosure is not limited to the particular implementations described above and is capable of numerous rearrangements, modifications, and substitutions without departing from the scope of the disclosure.

## Claims

1. An orthopedic fixation fastener for fixing a suture, comprising:
a fixation fastener body (1) comprising:
a central portion (11),
a first fixing portion, (12) and
a second fixing portion (13), wherein the first fixing portion (12) and the second fixing portion (13) are connected to opposite sides of the central portion (11), and the central portion (11), the first fixing portion (12), and the second fixing portion (13) as a whole define a top surface (14) and a bottom surface (15); wherein each of the first fixing portion (12) and the second fixing portion (13) comprises a first suture hole (121) and a second suture hole (131) extending through the top surface (14) and the bottom surface (15) for a corresponding suture to pass through; and
a fixator (2) comprising a fixing structure (21) and being detachably connected to the fixation fastener body (1) via the fixing structure (21), wherein when the fixator (2) is connected to the fixation fastener body (1), at least one suture is compressed and fixed between the fixator (2) and the fixation fastener body (1).

2. The orthopedic fixation fastener of claim 1, wherein the fixing structure (21) comprises a first hook structure (211) bent at a first angle toward the fixation fastener body (1), the first hook structure (211) being detachably hooked and fixed to an engaging structure (111) in the central portion (11).

3. The orthopedic fixation fastener of claim 1, wherein the fixing structure (21) comprises a first hook structure (211) bent at a first angle toward the fixation fastener body (1) and a second hook structure (212) bent at a second angle toward the fixation fastener body (1), the first hook structure (211) and the second hook structure (212) being detachably hooked and fixed to a set of engaging structures (111) in the central portion (11) correspondingly.

4. The orthopedic fixation fastener of claim 3, wherein the first angle is greater than the second angle.

5. The orthopedic fixation fastener of claim 1, wherein when the fixator (2) is connected to the fixation fastener body (1), an accommodating space (3) having a width less than or equal to the diameter of the at least one suture is formed between the fixator (2) and the fixation fastener body (1).

6. The orthopedic fixation fastener of claim 1, wherein the fixing structure (21) comprises a plurality of post structures (213), and the fixation fastener body (1) further comprises a plurality of corresponding hole structures (112), wherein the plurality of post structures (213) is detachably engaged with the plurality of corresponding hole structures (112).

7. The orthopedic fixation fastener of claim 1, wherein the fixator (2) further comprises a third suture hole (22) for the corresponding suture to pass through.

8. The orthopedic fixation fastener of claim 1, wherein a part or an entirety of the central portion (11) of the fixation fastener body (1) is recessed from the top surface (14) toward the bottom surface (15) by a depth equal to or less than the diameter of the at least one suture to form a recessed structure (113).

9. The orthopedic fixation fastener of claim 8, wherein a part of the recessed structure (113) forms a directional groove (114) for accommodating the at least one suture.

10. The orthopedic fixation fastener of claim 1, wherein the fixator (2) further comprises a baffle structure (23), the baffle structure (23) being a protrusion extending from the fixator (2) toward the fixation fastener body (1) by a length that is less than the diameter of the at least one suture, and disposed on a side of the central portion (11) that is not connected to the first fixing portion (12) and the second fixing portion (13) when the fixator (2) is engaged with the fixation fastener body (1).
